# EUROPÄISCHE PATENTANMELDUNG

(11) **EP 1 574 163 A1**
(43) Veröffentlichungstag der Anmeldung: **14.09.2005**
(21) Anmeldenummer: 05003266.3
(22) Anmeldetag: 16.02.2005
(51) Int. Cl.: A61B 1/31, A61B 1/32

(54) **Analspreizer**

(30) Priorität: 12.03.2004 DE 102004012109
(71) Anmelder: Fehling AG, 4153 Reinach BL 1 (CH)
(72) Erfinder: Fehling, Guido, 63791 Karlstein (DE)
(74) Vertreter: Mussgnug, Bernd

(57) **Zusammenfassung**

Ein Analspreizer weist vier Spreizblätter (48) auf, die unter 90° gegeneinander versetzt radial auseinander bewegt werden können. Hierzu werden die vier Spreizblätter (48) mit Schlitten (30) in radialen Führungsschlitzen (14) einer Grundplatte (10) geführt. Zur Bewegung der Schlitten (30) durchgreifen Führungsbolzen (36) die radialen Führungsschlitze (14) der Grundplatte 10 und spiralförmige Betätigungsschlitze (24) einer Rotorplatte (20), die gegenüber der Grundplatte (10) verdrehbar ist.

## Beschreibung

Die Erfindung betrifft einen Analspreizer gemäß dem Oberbegriff des Anspruchs 1.

Um den Analkanal für ärztliche Eingriffe und Untersuchungen aufzuweiten, werden Analspreizer eingesetzt. Diese Analspreizer weisen Spreizblätter auf, die in den Analkanal eingeführt werden und dann zur Erweiterung des Analkanals auseinander bewegt werden.

In einer bekannten Ausführung sind zwei Spreizblätter vorgesehen, die mittels eines Scherengriffes auseinander gespreizt werden. Damit ist nur ein verhältnismäßig geringes Aufweiten des Analkanals möglich. Weiter ist ein Analspreizer mit zwei Spreizblättern bekannt, bei welchem das eine Spreizblatt auf einer an dem anderen Spreizblatt angebrachten Führungsschiene verschiebbar ist. Damit können die Spreizblätter zwar über einen großen Weg auseinander bewegt werden, das Aufweiten des Analkanals erfolgt jedoch nur in einer diametralen Richtung, so dass der Zugangsquerschnitt nicht optimal vergrößert wird. Schließlich ist ein Analspreizer bekannt, bei welchem zwei Spreizblätter mittels eines Scherengriffes auseinander bewegt werden können und ein drittes Spreizblatt in einer zur Schwenkebene des Scherengriffes senkrechten Ebene angeordnet ist. Die drei Spreizblätter erzeugen einen größeren Zugangsquerschnitt des aufgeweiteten Analkanals. Der Zugangsquerschnitt ist jedoch auch hierbei asymmetrisch und im Wesentlichen durch die diametrale Spreizbewegung des Scherengriffes bestimmt.

Der Erfindung liegt die Aufgabe zugrunde, einen Analspreizer zu schaffen, der einen verbesserten Zugang für ärztliche Untersuchungen und Eingriffe ermöglicht.

Diese Aufgabe wird erfindungsgemäß gelöst durch einen Analspreizer mit den Merkmalen des Anspruchs 1.

Vorteilhafte Ausführungen und Weiterbildungen der Erfindungen sind in den Unteransprüchen angegeben.

Bei dem erfindungsgemäßen Analspreizer sind die Spreizblätter an einer Grundplatte radial verschiebbar geführt. Dadurch ist es möglich, eine größere Anzahl von Spreizblättern symmetrisch um eine mittige freie Öffnung der Grundplatte anzuordnen und dadurch den Analkanal symmetrisch mit optimalem Querschnitt aufzuweiten. Werden drei Spreizblätter verwendet, die dann im Winkel von 120° gegeneinander versetzt radial verschiebbar sind, so ergibt sich eine dreieckige Aufweitung des Analkanals. Eine optimale Ausführung ergibt sich mit vier Spreizblättern, die jeweils in um 90° gegeneinander versetzten Führungen radial verschiebbar sind. Die vier Spreizblätter ergeben einen in etwa quadratischen Zugangsquerschnitt des aufgeweiteten Analkanals, der dem Arzt eine große Bewegungsfreiheit ermöglicht. Mehr als vier Spreizblätter sind erfindungsgemäß möglich, bringen aber keine wesentliche Verbesserung im Zugangsquerschnitt, führen jedoch zu einer größeren Abdeckung der Wand des Analkanals durch die Spreizblätter.

In einer vorteilhaften Ausführung sind die Spreizblätter mittels Schlitten in den radialen Führungen geführt. Damit ergibt sich zum einen eine stabile, belastbare und verkippsichere Führung der Spreizblätter. Es ist insbesondere auch möglich, die Spreizblätter an dem Schlitten auswechselbar zu befestigen, wobei insbesondere eine Rastverbindung vorgesehen sein kann. Es ist dadurch möglich, den Analspreizer mit einem Grundinstrument auszubilden, welches in Verbindung mit dem jeweiligen Anwendungsfall angepassten Spreizblättern ausgestattet werden kann.

In einer bevorzugten Ausführung werden die Spreizblätter und insbesondere die die Spreizblätter tragenden Schlitten in den radialen Führungen durch eine Rotorplatte bewegt. Die Spreizblätter bzw. die diese tragenden Schlitten weisen Führungsbolzen auf, die die radialen Führungen der Grundplatte und diese radialen Führungen schneidende spiralförmige Betätigungsschlitze der Rotorplatte durchgreifen. Werden die Grundplatte und die Rotorplatte konzentrisch gegeneinander verdreht, so wandern die Kreuzungspunkte der radialen Führungsschlitze und der spiralförmigen Betätigungsschlitze, in denen sich die Führungsbolzen befinden, radial nach außen und bewegen die Spreizblätter radial auseinander. Dadurch können die Spreizblätter exakt symmetrisch zueinander und mit feinfühlig dosierbarem Kraftaufwand auseinander gespreizt werden.

In einer bevorzugten Ausführung können die Grundplatte und di Rotorplatte mittels einer Spanneinrichtung in der jeweiligen Drehstellung miteinander verklemmt werden, um die Spreizblätter in der jeweiligen Spreizstellung zu arretieren.

Um den Analspreizer möglichst schmerzfrei und ohne Gewebeverletzungen einführen zu können, ist vorzugsweise ein Mandrin vorgesehen, der mittig in die Grundplatte eingesetzt werden kann. Der Mandrin ragt distal über die Grundplatte hinaus und weist ein distales stumpfkonisches Endstück auf. Wenn die Spreizblätter in ihre radial innerste Position geschoben sind, greifen die distalen freien Enden der Spreizblätter hinter der proximalen Rand des konischen Endstückes und werden durch das Endstück abgedeckt, wenn der Mandrin mit den anliegenden Spreizblättern in den Analkanal eingeführt wird. Sobald die Spreizblätter im Analkanal positioniert sind, werden diese radial auseinander bewegt, so dass der Mandrin mit seinem konischen Endstück nach proximal herausgezogen werden kann.

In einer zweckmäßigen Ausführung weist der Mandrin einen axialen Stift auf, der das distale Endstück trägt. Dieser Stift ist axial verstellbar in einem proximalen Mandringriff gelagert. Dadurch kann die axiale Position des Endstückes in Bezug auf die Grundplatte eingestellt und der Länge der jeweils verwendeten Spreizblätter angepasst werden.

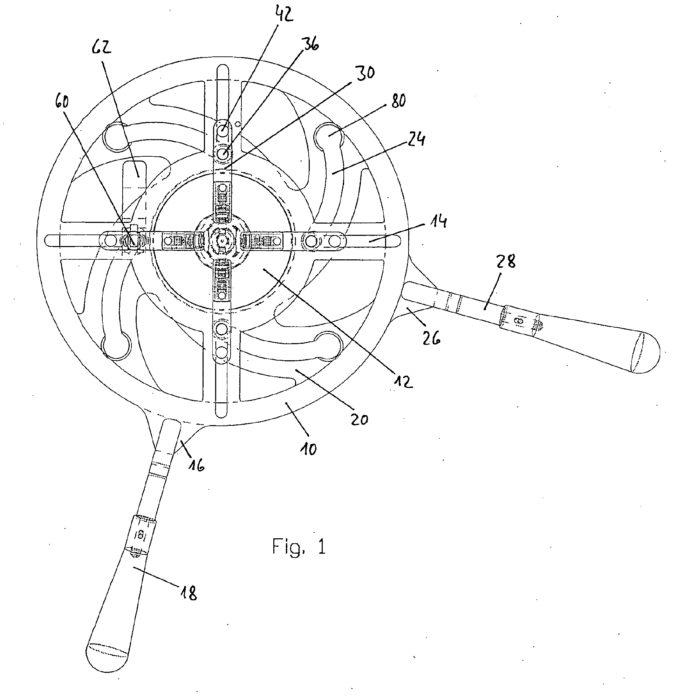

Im Folgenden wird die Erfindung anhand eines in der Zeichnung dargestellten Ausführungsbeispiels näher erläutert. Es zeigen
- Fig. 1: eine axiale Draufsicht auf den Analspreizer von der distalen Seite,
- Fig. 2: eine Seitenansicht des Analspreizers,
- Fig. 3: einen Axialschnitt längs der Schnittlinie A-A in Figur 1,
- Fig. 4: eine Draufsicht auf die Grundplatte,
- Fig. 5: eine Draufsicht auf die Rotorplatte,
- Fig. 6: eine perspektivische Ansicht eines Spreizblattes mit Schieber,
- Fig. 7: einen Axialschnitt des Spreizblattes mit Schieber gemäß Figur 6 und
- Fig. 8: einen Axialschnitt durch den Mandrin.

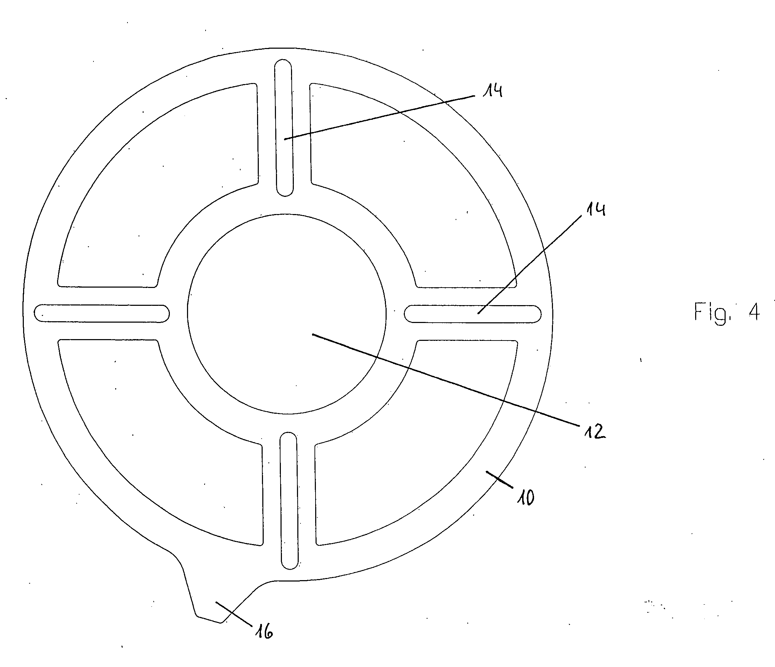

Der Analspreizer weist eine Grundplatte 10 auf, die in Figur 4 als Einzelteil gezeigt ist. Die Grundplatte 10 hat die Form einer Kreisscheibe mit einer mittigen kreisförmigen freien Öffnung 12. Die Öffnung 12 bestimmt den freien Zugangsquerschnitt und weist beispielsweise einen Durchmesser von 60 mm auf. Außerhalb der Öffnung 12 verlaufen radiale Führungen, die als die Grundplatte 10 durchbrechende Führungsschlitze 14 ausgebildet sind. In dem dargestellten Ausführungsbeispiel sind vier Führungsschlitze 14 vorgesehen, die in gleicher Winkelteilung jeweils um 90° gegeneinander versetzt sind. Am Außenumfang weist die Grundplatte 10 einen radial abstehenden Lappen 16 auf, an welchem ein radial abstehender Griffhebel 18 befestigt, z.B. angeschweißt ist.

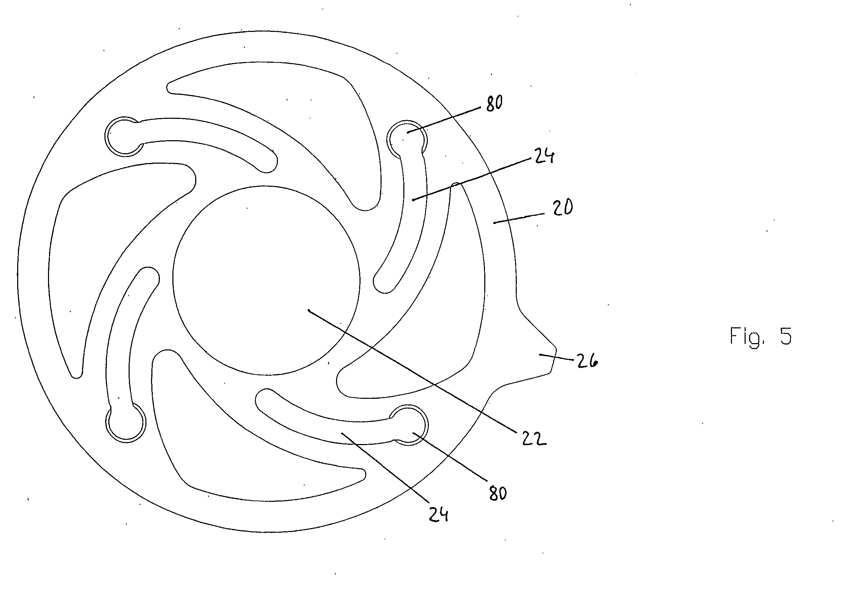

An der proximalen Fläche der Grundplatte 10 liegt eine Rotorplatte 20 an, die in Figur 5 als Einzelteil dargestellt ist. Die Rotorplatte 20 hat ebenfalls die Form einer Kreisscheibe, wobei ihr Außendurchmesser im Wesentlichen mit dem Außendurchmesser der Grundplatte 10 übereinstimmt. Mittig weist die Rotorplatte 20 ebenfalls eine freie Öffnung 22 mit kreisförmigen Querschnitt auf, deren Durchmesser wenigstens gleich dem Durchmesser der mittigen Öffnung der Grundplatte 10 ist. Die Rotorplatte 20 ist außerhalb der Öffnung 22 von Betätigungsschlitzen 24 durchbrochen. Die Betätigungsschlitze 24 verlaufen spiralförmig nach außen. Die Zahl und Anordnung der Betätigungsschlitze 24 entspricht den Führungsschlitzen 14 der Grundplatte 10. Im dargestellten Ausführungsbeispiel sind dementsprechend vier jeweils um 90° gegeneinander versetzte Betätigungsschlitze 24 vorgesehen. Das innere Ende der Führungsschlitze 14 und der Betätigungsschlitze 24 liegt auf demselben Radius und ebenso liegen die äußeren Enden der Führungsschlitze 14 und Betätigungsschlitze 24 auf demselben Radius. Auf Grund des spiralförmigen Verlaufes der Betätigungsschlitze 24 ist deren äußeres Ende gegenüber dem inneren Ende jeweils um etwa 45° versetzt. Die Rotorplatte 20 weist an ihrem Außenumfang ebenfalls einen radial abstehenden Lappen 26 auf, an welchem ein radial abstehender Griffhebel 28 befestigt, z.B. angeschweißt ist.

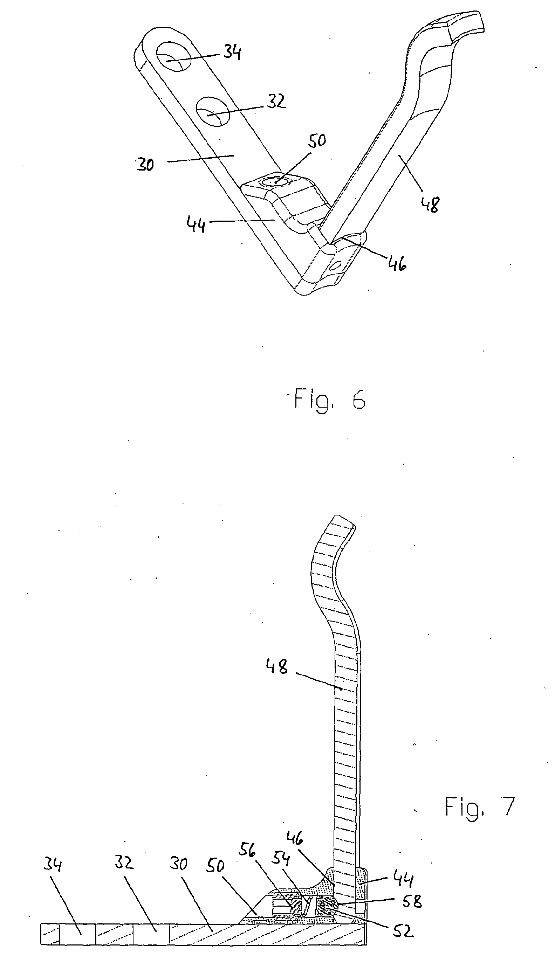

Distal auf der Grundplatte 10 sitzen jeweils den Führungsschlitzen 14 zugeordnete Schlitten 30, die in den Figuren 6 und 7 als Einzelteil dargestellt sind. Die Schlitten 30 haben die Form einer flachen länglichen Platte, die in radialer Richtung parallel auf den Führungsschlitzen 14 aufliegt. Die Schlitten 30 weisen zwei in radialer Richtung beabstandete Bohrungen 32 und 34 auf. Der Durchmesser der Bohrungen 32 und 34 entspricht der Breite der Führungsschlitze 14. In die radial innere Bohrung 32 ist Führungsbolzen 36 fest eingepasst. Der Führungsbolzen 36 durchsetzt die Bohrung 32, den Führungsschlitz 14 der Grundplatte 10 und den jeweiligen Betätigungsschlitz 24 der anliegenden Rotorplatte 20. Auf der proximalen Seite übergreift der Führungsbolzen 36 den Betätigungsschlitz 24 mit einem verbreiterten Kopf 38, wodurch die Rotorplatte 20 an der Grundplatte 10 gehalten wird. In die radial äußere Bohrung 34 des Schlittens 30 ist ein Lagerzapfen 42 eingesetzt, der unverlierbar in dem Schlitten 30 gehalten ist und in den Führungsschlitz 14 eingreift.

Auf dem radial mittleren Ende des Schlittens 30 ist ein Block 44 ausgebildet. Der Block 44 weist eine Aufnahmetasche 46 auf, die nahe am radial inneren Ende des Schlittens 30 angeordnet ist, die von der distalen Seite senkrecht zu dem Schlitten 30 in den Block 44 führt und die die Querschnittsform eines senkrecht zur Längsrichtung 30 verlaufenden Schlitzes hat. In die Aufnahmetasche 46 sind jeweils Spreizblätter 48 auswechselbar eingesetzt. Hierzu ist in dem Block 44 eine Bohrung 50 vorgesehen, die parallel zur Oberfläche und Längserstreckung des Schlittens 30 verläuft und bis in die Aufnahmetasche 46 reicht. In diese Bohrung 50 ist eine Rastkugel 52 eingesetzt, die mit ihrem Umfang in die Aufnahmetasche 46 hineinragt und durch eine Druckfeder 54 vorgespannt ist. Die Druckfeder stützt sich mit ihrem von der Rastkugel 52 abgewandten Ende an einer Einstellschraube 56 ab, die in ein Innengewinde der Bohrung 50 eingedreht ist und mittels derer die Vorspannung der Druckfeder 54 und damit der Rastkugel 52 eingestellt werden kann. Das Spreizblatt 48 weist an seinem in die Aufnahmetasche 46 eingreifenden Ende eine Rastvertiefung 58 auf, in welche die Rastkugel 52 federnd einschnappt, um das Spreizblatt 48 in der Aufnahmetasche 46 zu arretieren. Auf diese Weise kann das Spreizblatt 48 durch einfaches Einschieben in die Aufnahmetasche 46 an dem Schlitten 30 verriegelt werden. Ebenso kann das Spreizblatt 48 wieder von dem Schlitten 30 getrennt werden, im dem das Spreizblatt 48 gegen die Rastkraft der Rastkugel 52 herausgezogen wird.

Werden die Grundplatte 10 und die Rotorplatte 20 mittels der Griffhebel 18 und 28 gegeneinander verdreht, so wandern die Kreuzungspunkte der radialen Führungsschlitze 14 und der spiralförmigen Betätigungsschlitze 24 radial. Da die Führungsbolzen 36 der Schlitten 30 in diesen Kreuzungspunkten sowohl den jeweiligen Führungsschlitz 14 als auch den jeweiligen Betätigungsschlitz 24 durchsetzen, verschieben sich damit auch die Führungsbolzen 36 radial in den Führungsschlitzen 14. Da die Schlitten 30 mit den Führungsbolzen 36 und dem Lagerzapfen 42 jeweils in den Führungsschlitzen 14 geführt sind, werden die Schlitten 30 durch die gegenseitige Verdrehung von Grundplatte 10 und Rotorplatte 20 radial entlang der Führungsschlitze 14 bewegt. Damit können die distal abstehenden Spreizblätter 48 der vier Schlitten 30 radial gegeneinander und auseinander bewegt werden.

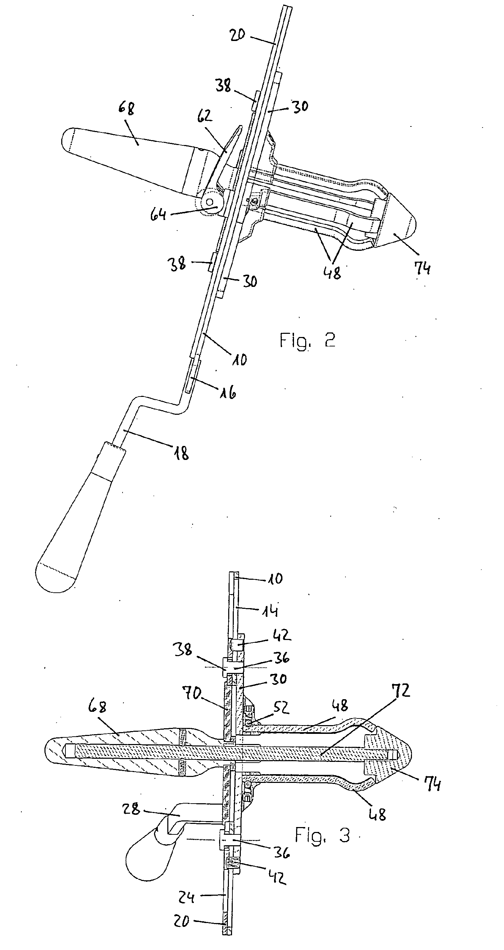

Bei einem der Schlitten 30 ist der Führungsbolzen 36 zusätzlich zu seiner Führungsfunktion auch als Spannbolzen 60 ausgebildet. An dem proximal über die Rotorplatte 20 hinaus ragenden Ende des Spannbolzens 60 ist anstelle des Kopfes 38 ein Exzenterhebel 62 um eine zur Achse des Spannbolzens senkrechte Achse schwenkbar gelagert. Der Exzenterhebel 62 weist im Bereich des Spannbolzens 60 einen Exzenternocken 64 auf, der sich an der Oberfläche der Rotorplatte 20 abstützt. Wird der Exzenterhebel 62 mit seinem freien Hebelgriff von der Rotorplatte 20 abgeschwenkt, so gibt der Exzenternocken 64 den Spannbolzen 60 für ein geringes axiales Spiel frei. Auf Grund dieses axialen Spiels kann sich der Schlitten 30 bewegen und die Grundplatte 10 und die Rotorplatte 20 können gegeneinander verdreht werden. Wird der Exzenterhebel 62 gegen die Rotorplatte 20 in die Spannstellung geschwenkt, wie dies in Figur 2 gezeigt ist, so stützt sich der Exzenternocken 64 an der Rotorplatte 20 ab und zieht den Spannbolzen 60 axial in proximaler Richtung. Damit werden die Grundplatte 10 und die Rotorplatte 20 zwischen dem Schlitten 30 und dem Exzenternocken 64 des Exzenterhebels 62 geklemmt und verspannt, so dass die Rotorplatte 20 und die Grundplatte 10 nicht mehr gegeneinander verdreht werden können und die Spreizblätter 48 in ihrer jeweiligen Position arretiert sind.

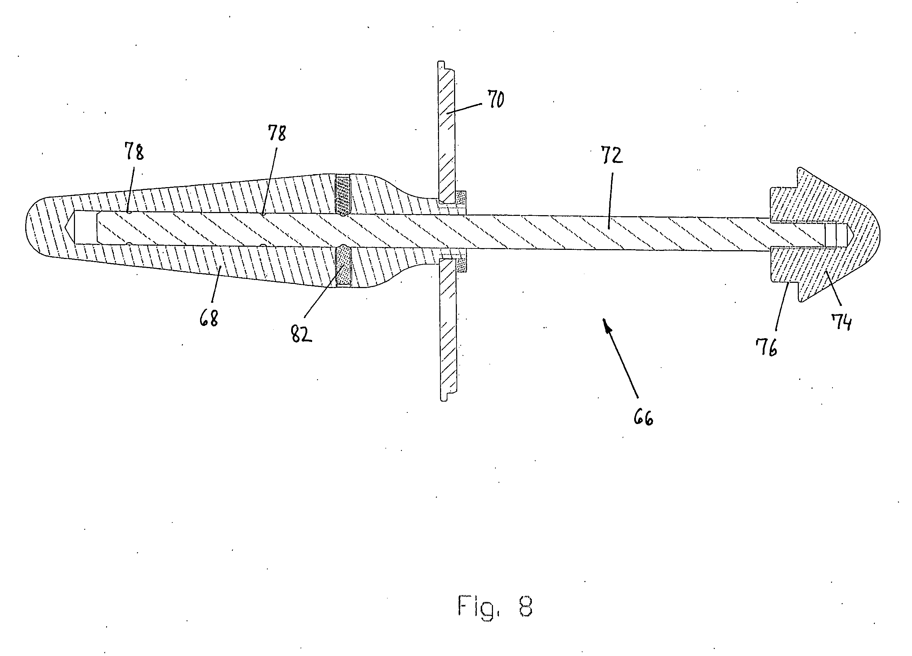

In die mittigen Öffnungen 12 der Grundplatte 10 und 22 der Rotorplatte 20 kann ein Mandrin 66 eingesetzt werden, der als Einzelteil in Figur 8 gezeigt ist.

Der Mandrin 66 weist einen proximalen Mandringriff 68 auf, der mittig in einer kreisscheibenförmigen Stützplatte 70 eingeschraubt ist. Der Außendurchmesser der Stützplatte entspricht dem Durchmesser der mittigen Öffnung 22 der Rotorplatte 20, so dass die Stützplatte 70 in diese Öffnung 22 eingesetzt werden kann, wobei sie sich mit einer Umfangsschulter am Umfang der Öffnung 22 abstützt. In den Mandringriff.68 ist axial ein Stift 72 eingesetzt, der distal aus dem Mandringriff 68 herausragt. Auf das distale Ende des Stiftes 62 ist ein Endstück 74 aufgesetzt, z.B. aufgeschraubt. Das Endstück 74 hat die Form eines Konus mit stumpfer distaler Spitze, der sich in proximaler Richtung erweitert. Am proximalen Ende weist das Endstück 64 eine Umfangsstufe 76 auf, die von dem proximalen Außenrand des Endstückes 74 radial übergriffen wird.

Der Stift 72 weist in seinem in den Mandringriff 68 eingreifenden Bereich axial beabstandete Rasteinstiche 78 auf. In diese Rasteinstiche können radial Raststücke 82, eingreifen, die in dem Mandringriff 68 aufgenommen sind. Dadurch ist es möglich, den Stift 72 in unterschiedlicher axialer Länge in dem Mandringriff 68 einzurasten. Dadurch wird festgelegt, wie weit das Endstück 74 distal von der Grundplatte 10 und den Schlitten 30 beabstandet ist, wenn der Mandrin 66 mit seiner Stützplatte 70 in die Rotorplatte 20 eingesetzt ist.

Für den Einsatz des Analspreizers werden die für den jeweiligen Anwendungsfall geeigneten Spreizblätter 48 in die Schlitten 30 eingerastet. Der Mandrin 66 wird mit seiner Stützplatte 70 in die Öffnung 22 der Rotorplatte 20 eingesetzt. Bei gelöstem Exzenterhebel 62 werden die Grundplatte 10 und die Rotorplatte 20 so verdreht, dass die Schlitten 30 radial nach innen geschoben werden. Die freien distalen Enden der Spreizblätter 48 legen sich dabei an die Umfangsstufe 76 des Endstückes 74 des Mandrins 66 an. Das Endstück 74 wird durch Verschieben und Einrasten des Stiftes 72 in dem Mandringriff 68 so eingestellt, dass die freien distalen Enden der Spreizblätter 48 in der Umfangsstufe 76 axial an dem Endstück 74 anliegen und durch dessen proximalen Außenumfang abgedeckt werden.

Nun kann der Mandrin 66 in den Analkanal des Patienten eingeführt werden. Durch das stumpfe konische Endstück 74 wird dabei ein möglichst schmerzfreies Einführen ermöglicht. Da das Endstück 74 die freien distalen Enden der Spreizblätter 48 übergreift, wird auch eine Gewebsverletzung beim Eindringen der Spreizblätter 48 in den Analkanal verhindert.

Sobald der Mandrin 66 mit den Spreizblättern 48 im Analkanal positioniert ist, werden die Grundplatte 10 und die Rotorplatte 20 mittels der Griffhebel 18 und 28 gegeneinander verdreht, sodass die Schlitten 30 mit den Spreizblättern 48 radial nach außen bewegt werden. Damit kommen die Spreizblätter 48 radial aus dem Bereich des Endstückes 74 und der Mandrin 66 mit dem Endstück 74 und der Stützplatte 70 kann nach proximal durch die Öffnungen 12 und 22 herausgezogen werden. Die Öffnungen 12 und 22 bilden nun den freien Zugangsquerschnitt des Analspreizers. Durch weiteres gegenseitiges Verdrehen von Grundplatte und Rotorplatte 20 werden nun die Schlitten 30 mit den Spreizblättern 48 weiter radial nach außen gefahren, um den Analkanal aufzuweiten. Ist der Analkanal weit genug aufgeweitet, so wird der Exzenterhebel 62 gegen die Rotorplatte niedergeschwenkt, um die Rotorplatte 20 und die Grundplatte 10 miteinander zu verklemmen und die Spreizblätter 48 in ihrer Lage zu arretieren.

Die Spreizblätter 48 weisen vorzugsweise eine geringe Breite von etwa 6 mm auf. Werden die Spreizblätter 48 auf den maximalen Abstand auseinandergefahren, der dem Durchmesser von 60 mm der Öffnungen 12 und 22 entspricht, so ergibt sich ein etwa quadratischer Zugangsquerschnitt des Analkanals mit einer Diagonalen von 60 mm, wobei die vier Spreizblätter 48 nur eine Fläche der Wand des Analkanals mit einer Breite von 24 mm in Umfangsrichtung bedecken. Es ist somit der größte Teil der Wand des Analkanals freiliegend, sodass Untersuchungen und Eingriffe in diesem Bereich des Analkanals durchgeführt werden können. Bei tiefer im Darmbereich liegenden Untersuchungen und Eingriffen ist eine größere Beweglichkeit der Instrumente möglich, da die freiliegenden und nicht durch die starren Spreizblätter 48 abgedeckten Bereiche der Wand des Analkanals den anliegenden Instrumenten nachgeben können.

In einer vorteilhaften Ausführung weisen die Betätigungsschlitze 24 jeweils Abschnitte 80 auf, die so weit verbreitert sind, dass der Kopf 38 der Führungsbolzen 36 durchtreten kann. Werden die Grundplatte 10 und die Rotorplatte 20 so gegeneinander verdreht, dass die Führungsbolzen 36 in die verbreiterten Abschnitte 80 der Betätigungsschlitze 24 kommen, so können die Führungsbolzen 36 durch die verbreiterten Abschnitte 80 heraus genommen werden, wodurch die Grundplatte 10 und die Rotorplatte 20 nicht mehr zusammengehalten sind. Die Grundplatte 10 und die Rotorplatte 20 können auf diese Weise ohne zu Hilfenahme von Werkzeugen zerlegt und wieder zusammengesetzt werden. Die verbreiterten Abschnitte 80 befinden sich vorzugsweise am radial äußeren Ende der Betätigungsschlitze 24, so dass die Zerlegung in einer Spreizstellung der Schlitten 30 und der Spreizblätter 48 erfolgt, die während der Benutzung des Analspreizers nicht erreicht wird.

### Bezugszeichen

- 10: Grundplatte
- 12: Öffnung
- 14: Führungsschlitze
- 16: Lappen
- 18: Griffhebel
- 20: Rotorplatte
- 22: Öffnung
- 24: Betätigungsschlitze
- 26: Lappen
- 28: Griffhebel
- 30: Schlitten
- 32: Bohrung
- 34: Bohrung
- 36: Führungsbolzen
- 38: Kopf
- 42: Lagerzapfen
- 44: Block
- 46: Aufnahmetasche
- 48: Spreizblatt
- 50: Bohrung
- 52: Rastkugel
- 54: Druckfeder
- 56: Einstellschraube
- 58: Rastvertiefung
- 60: Spannbolzen
- 62: Exzenterhebel
- 64: Exzenternocken
- 66: Mandrin
- 68: Mandringriff
- 70: Stützplatte
- 72: Stift
- 74: Endstück
- 76: Umfangsstufe
- 78: Rasteinstiche
- 80: verbreiterte Abschnitte
- 82: Raststück

## Patentansprüche

1. Analspreizer mit geführt auseinanderbewegbaren Spreizblättern,
**gekennzeichnet durch** eine Grundplatte (10) mit einer mittigen freien Öffnung (12), **durch** wenigstens drei außerhalb der Öffnung (12) im Winkel gegeneinander versetzt in der Grundplatte (10) angeordnete radial verlaufende Führungen (14), **durch** in den Führungen (14) radial verschiebbare Spreizblätter (48), die in distaler Richtung im Wesentlichen senkrecht von der Grundplatte (10) abstehen, und **durch** Betätigungsmittel (20, 24) zum radialen Verschieben der Spreizblätter (48) in den Führungen (14).

2. Analspreizer nach Anspruch 1,
**dadurch gekennzeichnet , dass**
die Führungen (14) in gleicher Winkelteilung gegeneinander versetzt angeordnet sind.

3. Analspreizer nach Anspruch 2,
**dadurch gekennzeichnet , dass**
vier Führungen (14) jeweils um 90° gegeneinander versetzt angeordnet sind.

4. Analspreizer nach einem der Ansprüche 1 bis 3,
**dadurch gekennzeichnet , dass**
in den Führungen (14) Schlitten (30) geführt sind und dass die Spreizblätter (48) auswechselbar in den Schlitten (30) gehalten sind.

5. Analspreizer nach Anspruch 4,
**dadurch gekennzeichnet , dass**
die Spreizblätter (48) einrastbar in den Schlitten (30) gehalten sind.

6. Analspreizer nach einem der vorhergehenden Ansprüche,
**dadurch gekennzeichnet , dass**
die Führungen als Führungsschlitze (14) in der Grundplatte (10) ausgebildet sind.

7. Analspreizer nach einem der vorhergehenden Ansprüche,
**dadurch gekennzeichnet , dass**
die Betätigungsmittel eine an der Grundplatte (10) anliegende Rotorplatte (20) aufweisen, dass die Grundplatte (10) und die Rotorplatte (20) konzentrisch gegeneinander drehbar sind, dass die Rotorplatte (20) jeweils die Führungsschlitze (14) der Grundplatte (10) schneidende spiralförmig verlaufende Betätigungsschlitze (24) aufweist und dass die Spreizblätter (48) mittels die Führungsschlitze (14) und die Betätigungsschlitze (24) durchgreifenden Führungsbolzen (36) verschiebbar sind.

8. Analspreizer nach Anspruch 7,
**dadurch gekennzeichnet , dass**
die Führungsbolzen (36) an den Schlitten (30) angeordnet sind.

9. Analspreizer nach Anspruch 7 oder 8,
**dadurch gekennzeichnet , dass**
die Grundplatte (10) und die Rbtorplatte (20) zur Arretierung der Spreizblätter (48) gegeneinander klemmbar sind.

10. Analspreizer nach Anspruch 9,
**dadurch gekennzeichnet , dass**
einer der Führungsbolzen (36) als Spannbolzen (60) ausgebildet ist, der mittels eines Spannelements (62, 64) axial bewegbar ist, um die Grundplatte (10) und die Rotorplatte (20) gegeneinander zu pressen und zu klemmen.

11. Analspreizer nach Anspruch 10,
**dadurch gekennzeichnet , dass**
das Spannelement ein an dem Spannbolzen (60) gelagerter Exzenterhebel (62) ist, der sich mit einem Exzenternocken (64) abstützt.

12. Analspreizer nach Anspruch 7,
**dadurch gekennzeichnet , dass**
die Grundplatte (10) und die Rotorplatte (20) als Kreisscheiben ausgebildet sind, die zur Betätigung von ihrem Außenrand radial abstehende Griffhebel (18, 28) aufweisen.

13. Analspreizer nach einem der vorhergehenden Ansprüche,
**dadurch gekennzeichnet , dass**
in die mittige Öffnung (12) der Grundplatte ein Mandrin (66) einsetzbar ist, der distal von der Grundplatte (10) absteht und an seinem distalen Ende ein konisches Endstück (74) aufweist, welches mit seinem proximalen Außenrand die distalen Enden der Spreizblätter (48) überdeckt, wenn die Spreizblätter (48) sich in ihrer radial inneren Stellung befinden.

14. Analspreizer nach Anspruch 13,
**dadurch gekennzeichnet , dass**
der Mandrin einen Stift (42) aufweist, der axial verstellbar in einem proximal von der Grundplatte (10) angeordneten Mandringriff (68) sitzt.

15. Analspreizer nach Anspruch 14,
**dadurch gekennzeichnet , dass**
der Stift (72) in dem Mandringriff (68) in definierten axialen Positionen einrastbar ist.

16. Analspreizer nach Anspruch 7,
**dadurch gekennzeichnet , dass**
die Führungsschlitze (14) und/oder die Betätigungsschlitze (24) verbreiterte Abschnitte (80) aufweisen, durch welche die Führungsbolzen (36) axial durchtreten können, um die Grundplatte (10) und die Rotorplatte (20) voneinander zu trennen.
